# EUROPEAN PATENT APPLICATION

(11) **EP 4 772 945 A1**
(43) Date of publication of application: **08.07.2026**
(21) Application number: 24859982.1
(22) Date of filing: 30.08.2024
(51) Int. Cl.: G03F 7/32, C07C 211/63, C23F 11/06, H01L 21/304, H01L 21/306, H01L 21/308

(54) **AQUEOUS ALKALI SOLUTION CONTAINING CORROSION INHIBITOR, AND METHOD FOR USING SAME AS LOW-TOXICITY AQUEOUS ALKALI SOLUTION**

(30) Priority: 01.09.2023 JP 2023142385; 08.02.2024 JP 2024017580; 10.05.2024 JP 2024077294; 17.07.2024 JP 2024114185
(71) Applicant: Tokuyama Corporation, Shunan-shi, Yamaguchi-ken 745-8648 (JP)
(72) Inventor: INOUE, Hiroshi, Shunan-shi, Yamaguchi 7458648 (JP); KAWABATA, Yuichiro, Shunan-shi, Yamaguchi 7458648 (JP)
(74) Representative: Watermeyer, Nicholas
(86) International application number: PCT/JP2024/031291
(87) International publication number: WO 2025/047957

(57) **Abstract**

Provided is an aqueous alkali solution containing (A) through (E). (A) is a quaternary ammonium ion represented by formula (1), (B) is a halide ion, (C) is a hydroxide ion, (D) is water, and (E) is a corrosion inhibitor. (In the formula, R¹, R², R³, and R⁴ are each independently a C1-16 alkyl group, and at least one of R¹, R², R³ and R⁴ is a C2-16 alkyl group.)

## Description

### Technical Field

The present invention relates to an aqueous alkali solution containing a corrosion inhibitor, and a method for using the same as a low toxic aqueous alkali solution. More specifically, the present invention relates to an aqueous alkali solution containing a predetermined quaternary ammonium ion, a halide ion, a hydroxide ion, water, and a corrosion inhibitor.

### Background Art

Quaternary ammonium compounds are used in phase transfer catalysts, surfactants, disinfectants, detergents, and the like. Among the quaternary ammonium compounds, tetramethylammonium hydroxide is particularly used as a kind of strongly basic organic alkali mainly in aqueous alkali solutions for semiconductors used in cleaning, etching, developers, and the like during semiconductor manufacturing.

For example, Patent Document 1 discloses a 2.35 wt.% aqueous tetramethylammonium hydroxide solution having a predetermined content of a metal ion and a halogen ion.

In addition, it is known that a quaternary ammonium compound exhibits alkalinity as an aqueous solution, and thus when a treatment liquid containing the quaternary ammonium hydroxide comes into contact with a metal, corrosion occurs. From the viewpoint of suppressing the corrosion of a metal, for example, Patent Document 2 proposes that a sugar or a sugar alcohol is added to tetramethylammonium hydroxide or choline to suppress the corrosion of aluminum.

### Prior Art Documents

### Patent Document

Patent Document 1: JP H04-226466 A
Patent Document 2: JP H04-48633 A

### Summary of Invention

### Technical Problem

However, the present inventors have focused on the fact that an aqueous tetramethylammonium hydroxide (hereinafter, also described as TMAH) solution is highly toxic and requires caution in handling.

Accordingly, an object of the present invention is to provide a low toxic aqueous alkali solution. Another object of the present invention is to provide a method for using a low toxic aqueous alkali solution for a specific application. Still another object of the present invention is to provide a method for producing a low toxic aqueous alkali solution.

### Solution to Problem

The present inventors have conducted intensive studies to yield a low toxic aqueous alkali solution. As a result, the present inventors have found that an aqueous alkali solution containing a specific quaternary ammonium ion, a halide ion, a hydroxide ion, and water has low toxicity, and that it is possible to suppress corrosion of a metal even in a case where a corrosion inhibitor is added to the low toxic aqueous organic alkali solution and the resultant is used for a specific application, thereby completing the present invention.

That is, the configuration of the present invention is as follows.
Aspect 1: An aqueous alkali solution containing the following (A) to (E):
   (A) a quaternary ammonium ion represented by Formula (1) below;
   (B) a halide ion;
   (C) a hydroxide ion;
   (D) water; and
   (E) a corrosion inhibitor, 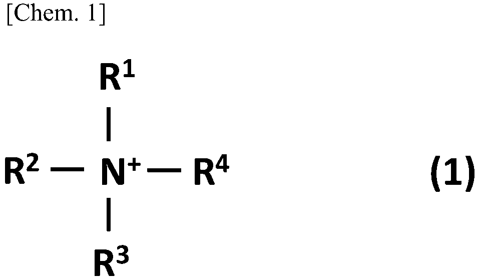 where R¹, R², R³, and R⁴ are each independently an alkyl group having carbon number from 1 to 16, with the proviso that the carbon number of one or more alkyl groups of R¹, R², R³, and R⁴ is from 2 to 16.
Aspect 2: The aqueous alkali solution according to Aspect 1, wherein R¹, R², R³, and R⁴ in Formula (1) are not all the same alkyl group.
Aspect 3: The aqueous alkali solution according to Aspect 1 or 2, wherein R¹, R², and R³ in Formula (1) are methyl groups, and R⁴ in Formula (1) is an alkyl group having carbon number from 2 to 16.
Aspect 4: The aqueous alkali solution according to any one of Aspects 1 to 3, wherein the halide ion (B) is a chloride ion or a bromide ion.
Aspect 5. The aqueous alkali solution according to any one of Aspects 1 to 4, wherein a part by mass of the halide ion is 0.0000001 to 0.01 parts by mass when a mass of the hydroxide ion in the aqueous alkali solution for semiconductors is 1 part by mass.
Aspect 6: The aqueous alkali solution according to any one of Aspects 1 to 5, wherein an LD50 value converted assuming that a hydroxide of the quaternary ammonium ion is taken as 100 mass%, in a toxicity test using a rat based on an acute oral toxicity test in accordance with OECD Test Guideline 420, is more than 50 mg/kg.
Aspect 7: The aqueous alkali solution according to any one of Aspects 1 to 6, wherein an EC50 value converted assuming that a hydroxide of the quaternary ammonium ion is taken as 100 mass%, in aDaphnia sp. acute immobilisation test in accordance with OECD Test Guideline 202, is more than 3 mg/L.
Aspect 8: The aqueous alkali solution according to any one of Aspects 1 to 7, further containing one or more compounds selected from the group consisting of an alcohol and an amine.
Aspect 9: The aqueous alkali solution according to any one of Aspects 1 to 8, further containing a surfactant, wherein a concentration of the surfactant in the aqueous alkali solution is less than 100 mass ppm.
Aspect 10: The aqueous alkali solution according to any one of Aspects 1 to 9, wherein the corrosion inhibitor (E) is one or more selected from the group consisting of an azole, an alkyne compound, and an aromatic hydroxy compound.
Aspect 11: A method for using the aqueous alkali solution according to any one of Aspects 1 to 10 as a low toxic aqueous alkali solution for semiconductors.
Aspect 12: A method for producing the aqueous alkali solution according to any one of Aspects 1 to 10, the method including an electrolysis step of electrolyzing an aqueous quaternary ammonium halide solution containing the following (A), (B), and (D), and a step of adding (E) a corrosion inhibitor to the aqueous solution obtained in the electrolysis step:
   (A) a quaternary ammonium ion represented by Formula (1) below;
   (B) a halide ion; and
   (D) water 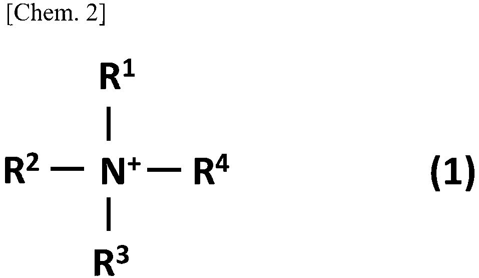
   where R¹, R², R³, and R⁴ are each independently an alkyl group having carbon number from 1 to 16, with the proviso that the carbon number of one or more alkyl groups of R¹, R², R³, and R⁴ is from 2 to 16.
Aspect 13: The method for producing the aqueous alkali solution according to Aspect 12, the method further including a preparation step of preparing the aqueous quaternary ammonium halide solution before the electrolysis step. wherein
   the preparation step is a step of reacting a trialkylamine with an alkyl halide in ultrapure water to yield the aqueous quaternary ammonium halide solution.

### Effects of Invention

According to the present invention, there is provided a low toxic aqueous alkali solution containing a corrosion inhibitor. As a result, an aqueous alkali solution is provided, which is highly safe in use and leads to a low environmental load. A method for using the low toxic aqueous alkali solution in a specific application is also provided. In addition, a method for producing a low toxic aqueous alkali solution is provided.

### Brief Description of Drawings

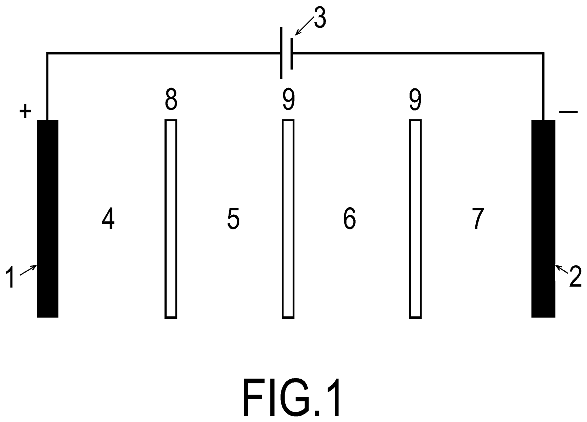

FIG. 1 is an explanatory view illustrating an electrolytic cell.

### Description of Embodiments

Embodiments of the present invention are described in detail below, but as long as the gist of the present invention is observed, the present invention is not limited to the details described below. In addition, the present invention can be modified and implemented in any manner that does not depart from the gist of the present invention.

In the present specification, a numerical range expressed using the term "to" refers to a range including the numerical values described before and after "to" as the lower limit and the upper limit, respectively. Thus, the expression "A to B" means A or more and B or less. Further, when numerical ranges are described in a stepwise manner, the upper limit and the lower limit of each numerical range can be optionally combined.

An aqueous alkali solution according to the present invention contains the following (A) to (E):
(A) a quaternary ammonium ion represented by Formula (1) below;
(B) a halide ion;
(C) a hydroxide ion;
(D) water; and
(E) a corrosion inhibitor, 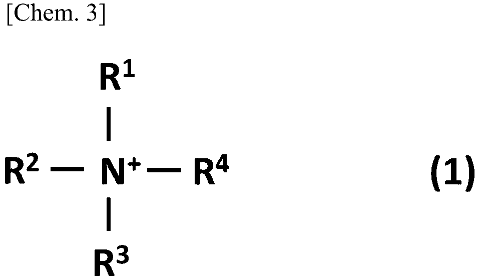 where R¹, R², R³, and R⁴ are each independently an alkyl group having carbon number from 1 to 16 (preferably from 1 to 8, and more preferably from 1 to 4), provided that one or more alkyl groups among R¹, R², R³, and R⁴ have carbon number from 2 to 16 (preferably from 2 to 8, and more preferably from 2 to 4).

The aqueous alkali solution contains the quaternary ammonium ion represented by Formula (1), and thus tends to have toxicity lower than that of an aqueous alkali solution using tetramethylammonium hydroxide.

In addition, the aqueous alkali solution contains the quaternary ammonium ion represented by Formula (1), and thus, when used as, for example, a cleaning liquid, an etching liquid, or a developer, the cleaning rate, the etching rate, or the development rate can be improved. When the carbon numbers of R¹ to R⁴ exceed the above range, the steric hindrance of the quaternary ammonium ion increases, and the etching rate and the development rate may decrease. When the carbon numbers of R¹ to R⁴ exceed the above range, a quaternary ammonium halide described below is less likely to be dissolved, which may make it difficult to manufacture the aqueous alkali solution.

In Formula (1), it is also preferable that R¹, R², R³, and R⁴ be not all the same alkyl group. In other words, quaternary ammonium ions represented by the following (1) to (3) are preferred:
(1) a quaternary ammonium ion in which any three of R¹, R², R³, and R⁴ are the same alkyl group, and the remaining one is an alkyl group different therefrom;
(2) a quaternary ammonium ion in which any two of R¹, R², R³, and R⁴ are the same alkyl group, and the remaining two are alkyl groups different therefrom (the remaining two can be the same or different); and
(3) a quaternary ammonium ion in which all of R¹, R², R³, and R⁴ are different alkyl groups.

In the case of the above (1), examples of the quaternary ammonium ion can include quaternary ammonium ions in which any three of R¹, R², R³, and R⁴ are the same alkyl group having carbon number from 1 to 16, the remaining one is an alkyl group having carbon number from 1 to 16, the first three and the remaining one are different alkyl groups, and either the first three or the remaining one has carbon number from 2 to 16. It is preferred that three of R¹, R², R³, and R⁴ in Formula (1) above be identical groups, and the remaining one be an alkyl group that has carbon number from 2 to 16 and is different from the three identical groups. In this case, the quaternary ammonium ion represented by Formula (1) has two types of alkyl groups. In this aspect, a quaternary ammonium ion in which any three of R¹, R², R³, and R⁴ are methyl groups and the remaining one alkyl group has carbon number from 2 to 16 is easy to serve as a surfactant. Among them, a quaternary ammonium ion in which the remaining one alkyl group has carbon number from 2 to 8 is more preferred, a quaternary ammonium ion in which the remaining one alkyl group has carbon number from 2 to 4 is still more preferred, and a quaternary ammonium ion in which the remaining one alkyl group has carbon number of 3 or 4 is most preferred.

In the case of the above (2), examples of the quaternary ammonium ion can include quaternary ammonium ions in which any two of R¹, R², R³, and R⁴ are the same alkyl group having carbon number from 1 to 16, the remaining two are alkyl groups having carbon number from 1 to 16, which are different from the first two, the remaining two are the same or different alkyl groups, and either the first two or the remaining two have carbon number from 2 to 16. In this case, the quaternary ammonium ion represented by Formula (1) has two or three types of alkyl groups. In this aspect, a quaternary ammonium ion in which any two of R¹, R², R³, and R⁴ are methyl groups, and the remaining two alkyl groups have carbon number from 2 to 16 is preferred. A quaternary ammonium ion in which any two of R¹, R², R³, and R⁴ are methyl groups is easy to serve as a surfactant. Among them, a quaternary ammonium ion in which the remaining two alkyl groups have carbon number from 2 to 8 is more preferred, a quaternary ammonium ion in which the remaining two alkyl groups have carbon number from 2 to 4 is still more preferred, and a quaternary ammonium ion in which the remaining two alkyl groups have carbon number of 3 or 4 is most preferred.

In the case of the above (3), examples of the quaternary ammonium ion can include quaternary ammonium ions in which all of R¹, R², R³, and R⁴ are different alkyl groups, and at least one of them has carbon number from 2 to 16. In this case, the quaternary ammonium ion represented by Formula (1) has four types of alkyl groups. In this aspect, a quaternary ammonium ion in which any one of R¹, R², R³, and R⁴ is a methyl group, and the remaining three alkyl groups have carbon number from 2 to 16 is preferred. A quaternary ammonium ion in which any one of R¹, R², R³, and R⁴ is a methyl group is easy to serve as a surfactant. Among them, a quaternary ammonium ion in which the remaining three alkyl groups have carbon number from 2 to 8 is more preferred, a quaternary ammonium ion in which the remaining three alkyl groups have carbon number from 2 to 4 is still more preferred, and a quaternary ammonium ion in which the remaining three alkyl groups have carbon number of 3 or 4 is most preferred.

Note that in a case where the quaternary ammonium ion represented by Formula (1) serves as a surfactant, it is possible to reduce an addition amount of a surfactant described below.

In Formula (1), all of R¹, R², R³, and R⁴ can be the same alkyl group. For example, all of R¹, R², R³, and R⁴ can be ethyl, propyl, or butyl.

Specific examples of the quaternary ammonium ion represented by Formula (1) include a tetraethylammonium ion, a tetrapropylammonium ion, a tetrabutylammonium ion, an ethyltrimethylammonium ion, a diethyldimethylammonium ion, a triethylmethylammonium ion, a propyltrimethylammonium ion, and a butyltrimethylammonium ion. One of the quaternary ammonium ions can be used, or a plurality thereof can be used. Examples of a preferred combination can include a combination of an ethyltrimethylammonium ion and a propyltrimethylammonium ion, and a combination of an ethyltrimethylammonium ion and a butyltrimethylammonium ion. These combinations can be further combined with another quaternary ammonium ion represented by Formula (1).

The halide ion is not particularly limited, and specific examples thereof include one or more selected from the group consisting of a chloride ion, a bromide ion, and an iodide ion. In addition, a chloride ion or a bromide ion is preferred.

A part by mass of the halide ion when the mass of the hydroxide ion in the aqueous alkali solution is 1 part by mass is not particularly limited, but is preferably from 0.0000001 to 0.01 parts by mass, more preferably from 0.0000001 to 0.001 parts by mass, and still more preferably from 0.0000001 to 0.0005 parts by mass. The parts by mass of the halide ion varies greatly depending on the method for producing the aqueous alkali solution, and a solution having a reduced amount of halide ion can be prepared by using a producing method by electrolysis in particular. The adjustment can be made by selecting the structure of an electrolytic cell and a cation exchange membrane used for electrolysis and changing conditions such as a current density.

When the part by mass of the halide ion is within the above range, a sufficient etching rate is maintained and surface roughness due to etching is suppressed in a case where the aqueous alkali solution is used as a treatment liquid for semiconductors such as an etching liquid for silicon, which is desirable.

A substrate used for silicon etching can be any of a single crystal, a polycrystal, and an amorphous, and an etching method is not particularly limited. As an example of the etching method, an aqueous alkali solution contains the quaternary ammonium ion represented by Formula (1), and a silicon substrate is immersed in the aqueous alkali solution for a predetermined time, for example, from 3 to 30 minutes, and preferably from 5 to 15 minutes, whereby etching can be performed. The temperature of the etching method can be, for example, from 25 to 90°C, and preferably from 30 to 80°C.

The part by mass of the halide ion in the aqueous alkali solution can be analyzed by ion chromatography, and analysis by a neutralization method is particularly effective when the aqueous alkali solution has a high concentration. Specifically, before the aqueous alkali solution is introduced into the anion ion chromatography, cations in the aqueous alkali solution are exchanged with hydrogen ions to convert hydroxide ions into water. Thus, the hydroxide ions can be removed. Further, the sensitivity is enhanced by concentration. By using this method, it is possible to measure the concentration of a low-concentration halide ion. This will be described in detail below.

The aqueous alkali solution contains a hydroxide ion. The fact that the aqueous alkali solution contains the quaternary ammonium ion represented by Formula (1) and the hydroxide ion can also be said to indicate that the aqueous alkali solution contains a quaternary ammonium hydroxide containing the quaternary ammonium ion represented by Formula (1) and the hydroxide ion. The aqueous alkali solution contains a quaternary ammonium hydroxide, and thus can be used as a cleaning liquid for various members and a treatment liquid during semiconductor manufacturing (treatment liquid for semiconductors).

Concentrations of the quaternary ammonium ion represented by Formula (1) and the hydroxide ion in the aqueous alkali solution are not particularly limited, and an aqueous alkali solution containing a quaternary ammonium hydroxide at a concentration of from 1.0 to 65.0 mass% is preferably manufactured. In a case of being used for a semiconductor application, for example, the quaternary ammonium hydroxide is more preferably used at a concentration of from 1.0 to 10.0 mass%, and is still more preferably used at a concentration of from 2.0 to 7.0 mass%. The concentration is more preferably from 1.0 to 3.0 mass%, and particularly preferably 2.38 mass%.

The quaternary ammonium hydroxide is preferably used at a molarity of from 0.10 to 0.50 mol/L, and is more preferably used at a molarity of from 0.15 to 0.35 mol/L.

Further, a high-concentration quaternary ammonium hydroxide can be diluted and used. With the high concentration, the volume during transportation can be reduced, which can suppress the transportation cost. Even when the aqueous alkali solution is contaminated when it is filled in a container or transported, the influence of contamination can be reduced by the extent of dilution, as compared with a case where the dilution is not performed. The concentration of the high-concentration quaternary ammonium hydroxide is preferably from 10.0 to 50.0 mass%, and more preferably from 20.0 to 40.0 mass%. The concentration of the quaternary ammonium hydroxide can be confirmed by neutralization titration with an acid or the like.

The concentration of the quaternary ammonium ion represented by Formula (1) in the aqueous alkali solution is preferably from 0.8 to 61.7 mass%. In addition, the concentration is more preferably from 0.8 to 9.5 mass%, and still more preferably from 1.2 to 6.7 mass%. The concentration of the quaternary ammonium ion in the high-concentration quaternary ammonium hydroxide before dilution is preferably from 8.0 to 47.5 mass%, and more preferably from 16.0 to 38.0 mass%.

The concentration of the hydroxide ion in the aqueous alkali solution is preferably from 0.05 to 10.6 mass%. In addition, the concentration is more preferably from 0.05 to 1.6 mass%, and still more preferably from 0.1 to 1.2 mass%. The concentration of the hydroxide ion in the high-concentration quaternary ammonium hydroxide is preferably from 0.5 to 8.0 mass%, and more preferably from 1.0 to 6.4 mass%.

The aqueous alkali solution contains water. A form of water is not particularly limited. Known water can optionally be used, and it is particularly preferable that the water be ultrapure water in which metal impurities are decreased. The content of water in the aqueous alkali solution for semiconductors is not particularly limited, but is preferably from 90.0 to 99.9 mass%, more preferably from 93.0 to 99.0 mass%, and still more preferably from 97.0 to 98.0 mass%.

The aqueous alkali solution contains a corrosion inhibitor. Although a metal such as aluminum or copper is corroded when it comes into contact with alkali, a corrosion rate is reduced by containing the corrosion inhibitor, and treatment can be performed without affecting the metal. In particular, even when the aqueous alkali solution contains the compound represented by Formula (1), the corrosion prevention effect can be obtained. As the corrosion inhibitor, one or more kinds selected from the group consisting of one or more kinds of azoles selected from the group consisting of triazole, thiazole, benzotriazole, and imidazole, alkyne compounds such as acetylene alcohol, aromatic hydroxy compounds, mercapto group-containing compounds, and the like can be used, and it is preferable to use one or more kinds selected from the group consisting of azoles, alkyne compounds, and aromatic hydroxy compounds, azoles are more preferable, and benzotriazole is still more preferable. One of these corrosion inhibitors can be used alone, or two or more thereof can be used in combination. The concentration in the aqueous alkali solution is preferably from 0.01 to 10 mass%, and can be more preferably from 0.1 to 3 mass%. By setting the concentration in such a range, the effect of preventing corrosion of a metal such as aluminum can be enhanced, and the treatment performance such as washing, peeling, and development can be maintained.

The aqueous alkali solution can contain one or more compounds selected from the group consisting of an alcohol and an amine. A concentration of the one or more compounds selected from the group consisting of an alcohol and an amine in the aqueous alkali solution is not particularly limited, but is preferably from 0.1 to 50 mass%, and more preferably from 0.5 to 40 mass%.

The alcohol is not particularly limited, and a known alcohol can be used. Examples of the alcohol include primary alcohols such as methanol, ethanol, and 1-propanol, secondary alcohols such as 2-propanol and 2-butanol, and tertiary alcohols such as 2-methyl-2-propanol. The number of hydroxyl groups in the alcohol is not particularly limited, and a monohydric alcohol can be used, or a dihydric or higher alcohol can be used. Among them, an alcohol having carbon number of 16 or less (more preferably carbon number from 2 to 8, and still more preferably carbon number from 2 to 4) is preferred from the viewpoint that the alcohol having a long chain increases the possibility of causing adsorption inhibition or the like during semiconductor treatment, for example.

The amine is not particularly limited, and a known amine can be used. Examples of the amine include primary amines such as methylamine, secondary amines such as dimethylamine, and tertiary amines such as trimethylamine. The number of amino groups in the amine is not particularly limited, and a monoamine can be used, or a diamine or higher amine can be used. Among them, an amine having carbon number of 16 or less (more preferably carbon number from 2 to 8, and still more preferably carbon number from 2 to 4) is preferred from the viewpoint that the amine having a long chain increases the possibility of causing adsorption inhibition or the like during treatment.

As described below, in a case using an aqueous alkali solution as a developer, a peeling liquid, or a cleaning liquid, specific examples of the amine can include one or more selected from the group consisting of N,N-diethylethanolamine, N,N-dimethylethanolamine, N-(2-aminoethyl)ethanolamine, N-methyldiethanolamine, N,N-dibutylethanolamine, N-methylethanolamine, ethanolamine, diethanolamine, triethanolamine, and propanolamine.

In a case where the aqueous alkali solution is used as the developer and the aqueous alkali solution contains these amines, the concentration thereof can be, for example, from 0.1 to 25 mass%. More preferably, the concentration can be from 1 to 5 mass%. By setting the concentration in such a range, the solubility of a resist can be enhanced, and the dissolution rate of the resist can be appropriately controlled.

In a case where the aqueous alkali solution is used as the peeling liquid or the cleaning liquid and the aqueous alkali solution contains these amines, the concentration thereof can be, for example, from 1 to 50 mass%. More preferably, the concentration can be from 10 to 40 mass%. By setting the concentration in such a range, the peelability of a resin layer or the like can be enhanced, and the peeling rate can be appropriately controlled.

The aqueous alkali solution can also contain a surfactant. The concentration of the surfactant in the aqueous alkali solution is preferably less than 100 mass ppm. The aqueous alkali solution does not need to contain a surfactant.

The quaternary ammonium ion represented by Formula (1) contained in the aqueous alkali solution is asymmetric with respect to the nitrogen atom as the center, and one or more alkyl groups of R¹, R², R³, and R⁴ have carbon number from 2 to 16, and thus, it is considered that the quaternary ammonium ion itself also serves as a surfactant. Specifically, a portion of the alkyl group having large carbon number becomes lipophilic, and a portion having small carbon number becomes hydrophilic. Accordingly, the quaternary ammonium ion represented by Formula (1) has a lipophilic moiety and a hydrophilic moiety in the ion structure, and is considered to serve as a surfactant. Thus, the aqueous alkali solution does not need to contain a surfactant at all, and even when it contains a surfactant, the concentration thereof can be as low as less than 100 mass ppm. The concentration of the surfactant is more preferably less than 50 mass ppm, still more preferably less than 30 mass ppm, and particularly preferably less than 10 mass ppm. When the aqueous alkali solution does not contain a surfactant, or when the concentration thereof is less than 100 mass ppm even if it contains a surfactant, for example, foaming of the aqueous alkali solution for semiconductors can be suppressed, and impurities derived from the surfactant can be suppressed. In addition, in a case where the quaternary ammonium hydroxide is recovered from the used aqueous alkali solution by separation, purification, or the like and is repeatedly used, it is necessary to add the surfactant again in a case where the surfactant is required as the aqueous alkali solution. However, in a case where the surfactant is not required to be contained, the recovered liquid can be used as it is as the aqueous alkali solution, and when a low concentration of the surfactant is sufficient, the amount of the surfactant to be added again can be suppressed, which is preferable. The lower limit of the surfactant is not particularly defined, but from the viewpoint of exhibiting the effect, the surfactant is preferably contained in an amount of 0.01 mass ppm or more, and more preferably 0.1 mass ppm or more.

The surfactant is a compound having a hydrophilic group and a hydrophobic group (lipophilic group) in one molecule, and examples thereof include a nonionic surfactant, an anionic surfactant, a cationic surfactant, and an amphoteric surfactant. As an example of the nonionic surfactant, for example, an acetylene glycol-based surfactant can be used. Examples of the acetylene glycol-based surfactant can include one or more selected from the group consisting of acetylene glycol, an ethylene oxide adduct of acetylene glycol, and acetylene alcohol. One of these compounds can be used alone, or two or more thereof can be used in combination.

In addition, in a case of using the aqueous alkali solution as a developer, a peeling liquid, or a cleaning liquid, it is preferable to add a nonionic surfactant to the aqueous alkali solution, and the concentration thereof is, for example, from 0.1 mass ppm to 10 mass%, and can be 1 mass ppm to 1 mass%. The upper limit of the concentration of the surfactant can be less than 100 mass ppm, less than 30 mass ppm, or less than 10 mass ppm as mentioned above.

Note that the surfactant is a compound different from the above-described compounds that can be contained in the aqueous alkali solution.

As evaluation of the toxicity of the aqueous alkali solution, a median lethal dose (LD50) value converted assuming that a hydroxide of a quaternary ammonium ion is taken as 100 mass%, in a toxicity test using a rat based on an acute oral toxicity test in accordance with OECD Test Guideline 420, is preferably more than 50 mg/kg. This makes the safety in use higher. In addition, in the GHS classification, in a case where the LD50 value is 50 mg/kg or less, it falls under Category 2, and regulations in storage, transportation, and the like are strict, and in a case where the LD50 value is 5 mg/kg or less, it falls under Category 1, and the regulations are further strict. Thus, the LD50 value is preferably more than 50 mg/kg. The upper limit of the LD50 value is not particularly limited, but is more preferably more than 50 mg/kg and 10000 mg/kg or less, and still more preferably more than 300 mg/kg and 10000 mg/kg or less.

The LD50 value can be adjusted by changing the type of the quaternary ammonium ion represented by Formula (1).

As evaluation of the toxicity of the aqueous alkali solution, a 50% effective concentration (EC50) value converted assuming that a hydroxide of a quaternary ammonium ion is taken as 100 mass%, in aDaphnia sp. acute immobilisation test in accordance with OECD Test Guideline 202, is preferably more than 3 mg/L. This makes the environmental load easy to be reduced. The EC50 value of TMAH, which is highly toxic and is likely to cause an environmental load, is 3 mg/L, and is preferably higher than 3 mg/L from the viewpoint of reducing an environmental load. The upper limit of the EC50 value is not particularly limited, but is more preferably more than 3 mg/L and 1000 mg/L or less, and still more preferably more than 10 mg/L and 1000 mg/L or less.

The EC50 value can be adjusted by changing the type of the quaternary ammonium ion represented by Formula (1).

In view of the toxicities obtained in the TG420 acute oral toxicity test and the TG202 Daphnia sp. acute immobilisation test of OECD, for example, a quaternary ammonium ion in which R¹, R², and R³ in Formula (1) are alkyl groups having carbon number of 1, and R⁴ is an alkyl group having carbon number from 2 to 4, a quaternary ammonium ion in which R¹, R², R³, and R⁴ are alkyl groups having carbon number of 2, a quaternary ammonium ion in which R¹, R², R³, and R⁴ are alkyl groups having carbon number of 3, a quaternary ammonium ion in which R¹, R², R³, and R⁴ are alkyl groups having carbon number of 4, or the like is preferably used. More specifically, an ethyltrimethylammonium ion, a propyltrimethylammonium ion, a butyltrimethylammonium ion, a tetraethylammonium ion, a tetrapropylammonium ion, and a tetrabutylammonium ion are preferred.

The aqueous alkali solution according to the present invention can be used as a low toxic aqueous alkali solution. That is, one aspect of the present invention is a method of using the aqueous alkali solution of the present invention as a low toxic aqueous alkali solution. The expression "using the aqueous alkali solution of the present invention as a low toxic aqueous alkali solution" means that the aqueous alkali solution of the present invention can be used for specific applications, specifically, various industrial applications, by utilizing the low toxicity thereof, as described below.

The low toxic aqueous alkali solution can be used, for example, for cleaning a metal member, and can also be used as a treatment liquid (treatment liquid for semiconductors) in semiconductor manufacturing, such as an etching liquid, a cleaning liquid, or a developer. That is, the low toxic aqueous alkali solution according to the present invention can be used as a low toxic aqueous alkali solution for manufacturing a semiconductor.

In a developer which is a specific example of the treatment liquid for semiconductors, tetramethylammonium hydroxide (TMAH) which is an organic base which has been generally used in the related art is highly toxic, and thus, regulations during storage and transportation are strict, and an environmental load is large in terms of waste liquid treatment after use. In contrast, the aqueous alkali solution according to the present invention has low toxicity, and thus, when a developer using the aqueous alkali solution is prepared and used, the above-mentioned problems are less likely to occur.

In a case of using the aqueous alkali solution of the present invention as a developer, the aqueous alkali solution can contain the above-mentioned surfactant and amine in addition to the above-mentioned components (A) to (E). As the concentrations of these components in the aqueous alkali solution, the ranges described above can be applied. In addition, in the case of using the aqueous alkali solution as a developer, as the concentration of the quaternary ammonium ion represented by Formula (1), the range described above can be exemplified.

In the case of using the aqueous alkali solution according to the present invention as a developer, various materials known to be developed by an alkaline compound can be used as the resist. The resist can be either a positive type or a negative type. The positive resist is not particularly limited, but examples thereof include (meth)acrylic resins such as polymethyl (meth)acrylate, urethane resins such as polyurethane, phenol resins such as a novolac resin, styrene resins such as a polyhydroxystyrene (PHS) resin, and resins having an aliphatic polycyclic skeleton. One of these resins can be used alone, or two or more thereof may be used in combination.

The negative resist is not particularly limited, but examples thereof include polyvinyl cinnamate and a mixture of a resin having an unsaturated carbon bond and an azide compound.

Note that in a case of using the aqueous alkali solution according to the present invention as a peeling liquid, it is suitably used for peeling a resin layer during semiconductor substrate preparation or the like, and a high peeling effect and a sufficient anticorrosion effect on metal wiring (particularly, Al-based and Cu-based) can be simultaneously realized.

In a case of using the aqueous alkali solution according to the present invention as a cleaning liquid, wettability to foreign matters on a semiconductor substrate is enhanced, and an excellent cleaning effect and a sufficient anticorrosion effect on metal wiring (particularly, Al-based and Cu-based) can be simultaneously realized.

A method for producing an aqueous alkali solution is not particularly limited, but from the viewpoint of suppressing the halide ion concentration, it is preferable to add the corrosion inhibitor (E) to an aqueous solution obtained by electrolyzing an aqueous quaternary ammonium halide solution, thereby producing the aqueous alkali solution. The method preferably includes an electrolysis step of electrolyzing the aqueous quaternary ammonium halide solution. Specifically, the method preferably includes an electrolysis step of electrolyzing an aqueous quaternary ammonium halide solution containing the following (A), (B), and (D). (A), (B), and (D) as described above can be used:
(A) a quaternary ammonium ion represented by Formula (1) below;
(B) a halide ion; and
(D) water 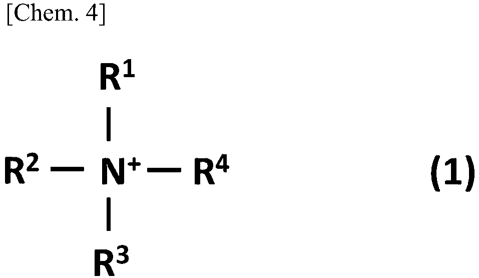
where R¹, R², R³, and R⁴ are each independently an alkyl group having carbon number from 1 to 16 (preferably from 1 to 8, and more preferably from 1 to 4), provided that one or more alkyl groups among R¹, R², R³, and R⁴ have carbon number from 2 to 16 (preferably from 2 to 8, more preferably from 2 to 4, and most preferably 3 or 4).

The manufacturing of the aqueous alkali solution by electrolysis can be performed by using an electrolytic cell in which one or more cation exchange membranes are disposed between an anode and a cathode and which is provided with a raw material chamber for supplying an aqueous quaternary ammonium halide solution as a raw material and a base chamber (cathode chamber) in which an aqueous quaternary ammonium hydroxide solution is produced. In the manufacturing method using this electrolytic cell, quaternary ammonium ions contained in the aqueous quaternary ammonium halide solution pass through the cation exchange membrane disposed on the cathode side during electrolysis. As a result, an aqueous quaternary ammonium hydroxide solution is produced in the base chamber.

The above example is an example in which one or more cation exchange membranes are disposed. However, an electrolytic cell in which a plurality of membranes selected from the group consisting of a cation exchange membrane, an anion exchange membrane, and a bipolar membrane (a composite membrane composed of a cation exchange membrane and an anion exchange membrane) are disposed can be used as long as an aqueous quaternary ammonium hydroxide solution can be produced.

At this time, a halide ion which is an anion of the quaternary ammonium halide moves to the anode side, but a part of the halide ions also moves to the cathode side by diffusion. Therefore, depending on the concentration of the liquid used as the raw material, about from 1 to several tens ppm of halide ions may be mixed into the aqueous quaternary ammonium hydroxide solution.

The method for producing an aqueous alkali solution can include, before the electrolysis step, a preparation step of preparing an aqueous quaternary ammonium halide solution to be used as a raw material in the electrolysis step.

The preparation step is not particularly limited. For example, an aqueous quaternary ammonium halide solution can be prepared by manufacturing an aqueous quaternary ammonium halide solution. A method for manufacturing the aqueous quaternary ammonium halide solution is not particularly limited. For example, from the viewpoint of productivity and quality, it is preferably a step of producing a quaternary ammonium halide by reacting a trialkylamine with an alkyl halide in ultrapure water.

The trialkylamine and the alkyl halide used as raw materials are not particularly limited as long as the quaternary ammonium ion represented by Formula (1) can be obtained. Specifically, among the alkyl groups contained in the trialkylamine and the alkyl groups contained in the alkyl halide, one or more alkyl groups can be alkyl groups having carbon number from 2 to 16 (preferably from 2 to 8, more preferably from 2 to 4, and most preferably 3 or 4), and the other alkyl groups can be alkyl groups having carbon number from 1 to 16 (preferably from 1 to 8, more preferably from 1 to 4, and most preferably 1).

The halide ion which is an anion of an alkyl halide can be the same as (B) described above.

### Examples

Hereinafter, to specifically describe the present invention, examples will be shown, but the present invention is not limited to these examples. The evaluations performed in Examples and Comparative Examples were determined by the following methods.

### (Reference Examples 1 to 13 and Comparative Example 1)

### [Aqueous Alkali Solution]

An aqueous quaternary ammonium hydroxide solution was prepared to have the concentration shown in Table 1, and various evaluations were performed as an aqueous alkali solution.
- Ethyltrimethylammonium hydroxide (ETMAH, made from raw materials)
- Diethyldimethylammonium hydroxide (DEDMAH, available from Sigma-Aldrich)
- Propyltrimethylammonium hydroxide (PTMAH, made from raw materials)
- Butyltrimethylammonium hydroxide (BTMAH, made from raw materials)
- Tetraethylammonium hydroxide (TEAH, available from Tokyo Chemical Industry Co., Ltd.)
- Tetrapropylammonium hydroxide (TPAH, available from Tokyo Chemical Industry Co., Ltd.)
- Tetrabutylammonium hydroxide (TBAH, available from Tokyo Chemical Industry Co., Ltd.)
- Hexadecyltrimethylammonium hydroxide (HTMAH, available from Tokyo Chemical Industry Co., Ltd.)
- Tetramethylammonium hydroxide (TMAH, available from Tokuyama Corporation)

### [Surfactant]

- SURFYNOL (registered trademark) 465 (available from Nisshin Chemical Industry Co., Ltd.)

### [Corrosion Inhibitor]

- Benzotriazole (available from Tokyo Chemical Industry Co., Ltd.)
- 2-butyne-1,4-diol (available from Tokyo Chemical Industry Co., Ltd.)
- Catechol (available from Tokyo Chemical Industry Co., Ltd.)

### [Amine]

- N,N-diethylethanolamine (available from Tokyo Chemical Industry Co., Ltd.)

### [Preparation of Aqueous Quaternary Ammonium Hydroxide Solution]

ETMAH, PTMAH, and BTMAH were prepared by the following procedure. An aqueous quaternary ammonium halide solution was produced from a trialkylamine and an alkyl halide as raw materials, and an aqueous quaternary ammonium hydroxide solution was produced by electrolysis using the aqueous quaternary ammonium halide solution as a raw material. Specifically, in the case of ETMAH, trimethylamine and ethyl chloride at a molar ratio of 1:1 were put into a reaction vessel containing ultrapure water, which were retained at a reaction temperature of 60°C to produce a 50 mass% aqueous ethyltrimethylammonium chloride solution. Trimethylamine and ethyl chloride, which were unreacted components, and ethanol, which was a by-product, were each removed under reduced pressure until the amounts thereof were each less than 100 ppm, and then ETMAH was produced using the electrolytic cell shown in FIG. 1. In FIG. 1, 1 denotes an anode, 2 denotes a cathode, 3 denotes a power supply, 4 denotes an anode chamber, 5 denotes a raw material chamber, 6 denotes an intermediate chamber, 7 denotes a cathode chamber, 8 denotes an anion exchange membrane, and 9 denotes a cation exchange membrane.

A platinum-plated nickel plate was used as the cathode, a platinum-plated titanium plate was used as the anode, two sheets of Nafion N324 (available from The Chemours Company) were used as the cation exchange membrane, and ASE (available from ASTOM CORPORATION) was used as the anion exchange membrane. Electrolysis was continuously carried out while gradually increasing the current density and finally maintaining the current density at 30 A/dm² and the temperature at 40°C, by circulating 0.5 N hydrochloric acid in the anode chamber, a 50 mass% aqueous ethyltrimethylammonium chloride solution in the raw material chamber between the anion exchange membrane and the cathode-side cation exchange membrane, and ultrapure water in the intermediate chamber between the cathode chamber and the two cation exchange membranes. During the electrolysis, the aqueous ethyltrimethylammonium chloride solution was replenished, and the concentration of ethyltrimethylammonium chloride in the raw material chamber was maintained at 40 mass% or more. When the concentration of ETMAH in the cathode chamber increased to 30 mass%, the electrolysis was terminated, resulting in the formation of an aqueous ETMAH solution. This aqueous ETMAH solution was diluted with ultrapure water and used in Reference Examples 1, 2, 12, 13, and 14, and Examples 1 and 10 at the concentrations shown in Table 1.

PTMAH and BTMAH were produced in the same manner as in the production of ETMAH except that propyl chloride and butyl chloride were used as alkyl halides, respectively.

ETMAH of Reference Example 3 was obtained in the same manner as in the production of ETMAH used in Reference Examples 1, 2, 12, 13, and 14 and Examples 1 and 10 except that the number of cation exchange membranes in FIG. 1 was reduced from two to one and an electrolytic cell without an intermediate chamber was used. In addition, the surfactant shown in Table 1 was added in Reference Examples 14 to 16 and Comparative Example 2, the corrosion inhibitor shown in Table 1 was added in Examples 1 to 13 and Comparative Example 3, and the amine shown in Table 1 was added in Example 13.

### [Evaluation Method]

### 1) Measurement of Halide Ion Concentration

In a case where the quaternary ammonium hydroxide concentration of the aqueous alkali solution was 5 mass% or less, the aqueous alkali solution was allowed to pass through an Ongard Cartridge IIH+ type 2.5 cc (available from Thermo Fisher Scientific Inc.) as it was, and in a case where the concentration exceeded 5 mass%, the aqueous alkali solution was diluted with ultrapure water to 5 mass% and allowed to pass through the Ongard Cartridge IIH+ type 2.5 cc. Thereafter, the liquid was recovered, and anion analysis of the recovered liquid was performed by an ion chromatograph (Integrion, available from Thermo Fisher Scientific Inc.) to determine a halide ion concentration.

### 2) Hydroxide Ion Concentration

One mL of each of the aqueous solutions prepared in Examples and Comparative Examples was subjected to neutralization titration with 0.1 mol/L hydrochloric acid using a Hiranuma automatic titrator (COM-1700, available from HIRANUMA). The hydroxide ion concentration of the aqueous alkali solution was determined from the liquid amount of 0.1 mol/L hydrochloric acid required for neutralization.

### 3) Acute Oral Toxicity

Data of the median lethal dose (LD50) in a toxicity test using a rat was collected. The aqueous quaternary ammonium hydroxide solutions for which no data was disclosed were subjected to a toxicity test based on the acute oral toxicity in accordance with OECD Test Guideline 420 to yield data.

The obtained median lethal dose was converted to 100 mass% of the aqueous quaternary ammonium hydroxide solution, and evaluation was performed based on the following criteria. In a case where two or more types of aqueous quaternary ammonium hydroxide solutions were mixed, the median lethal dose of each aqueous quaternary ammonium hydroxide solution was multiplied by the mixing ratio, and the sum of the resulting values was determined. The results are shown in Table 1.
A: Median lethal dose of more than 300 mg/kg
B: Median lethal dose of more than 50 mg/kg to 300 mg/kg or less
C: Median lethal dose of 50 mg/kg or less

When the alkali aqueous solution is evaluated as A or B, it can be said to be a low toxic aqueous alkali solution for semiconductors, and is preferably evaluated as A.

### 4) Ecotoxicity (Acute)

Data were collected for the 50% effective concentration (EC50) in the Daphnia sp. acute immobilisation test. The aqueous quaternary ammonium hydroxide solutions for which no data was disclosed were subjected to a toxicity test based on the Daphnia sp. acute immobilisation test in accordance with OECD Test Guideline 202 to yield data.

The obtained median lethal dose was converted to 100 mass% of the aqueous quaternary ammonium hydroxide solution, and evaluation was performed based on the following criteria. In a case where two or more types of aqueous quaternary ammonium hydroxide solutions were mixed, the median lethal dose of each aqueous quaternary ammonium hydroxide solution was multiplied by the mixing ratio, and the sum of the resulting values was determined. The results are shown in Table 1.
A: 50% effective concentration of more than 10 mg/L
B: 50% effective concentration of more than 3 mg/L to 10 mg/L or less
C: 50% effective concentration of 3 mg/L or less

The aqueous alkali solution evaluated as A or B can be said to be a low toxic aqueous alkali solution, and is preferably evaluated as A.

In addition, even when the ecotoxicity is evaluated as C, if the acute oral toxicity is evaluated as A to B, the aqueous solution can be evaluated as low toxicity.

### 5) Development Test

4-inch silicon wafers were prepared, and the surfaces thereof were washed with sulfuric acid-hydrogen peroxide (volume ratio: 4 : 1). The silicon wafers were baked on a hot plate at 200°C for 60 seconds. Then, a positive photoresist (novolac resin) was applied to the silicon wafers using a spinner to yield positive photoresist films each having a thickness of 3.5 µm. Next, these photoresist films were irradiated with g-, h-, and i-rays having a wavelength of 300 to 500 µm through a mask pattern, and then various aqueous alkali solutions were dropped thereon. The development time was set to 8 minutes or 12 minutes, and the photoresist films were left to stand at 23°C, then washed with ultrapure water, and dried to yield a 20 µm contact hole pattern.

The presence or absence of scum and the shape of the obtained resist pattern were confirmed by SEM, and evaluation was performed based on the following criteria. The results are shown in Table 2.
A: No scum is generated in both cases of the development time of 8 minutes and 12 minutes, and the side wall of the recessed portion of the pattern is upright
B: Scum is observed in a part of the recessed portion in the case of the development time of 8 minutes, but no scum is generated in the case of the development time of 12 minutes, and the side wall of the recessed portion of the pattern is upright
C: Scum is generated in both the cases of the development time of 8 minutes and 12 minutes, and/or the side wall of the recessed portion of the pattern is not upright

As the developer for a semiconductor, the aqueous alkali solution is preferably evaluated as A, and even the aqueous alkali solution for semiconductors evaluated as B can be used under appropriate conditions.

### 6) Corrosion Test

100 mL of the aqueous alkali solution was prepared. An aluminum piece having a size of 2 × 2 cm was immersed in the alkali solution at 25°C for 10 minutes, and then the aluminum piece was rinsed with pure water. A corrosion amount (dissolution amount) was determined from the mass change before and after the treatment, and the corrosion amount was divided by the immersion time in the aqueous alkali solution to determine a corrosion rate.

The obtained corrosion rate was evaluated based on the following criteria.
A: Corrosion rate of less than 1 nm/min
B: Corrosion rate of 1 nm/min or more and less than 10 nm/min
C: Corrosion rate of 10 nm/min or more

From the viewpoint of suppressing corrosion, the evaluation is preferably A.

**[Table 1-1]**

| | Quaternary ammonium hydroxide | | Quaternary ammonium hydroxide | | Surfactant | | Corrosion inhibitor | | Amine | |
|---|---|---|---|---|---|---|---|---|---|---|
| | Liquid type | mass % | Liquid type | mass% | Type | mass ppm | Liquid type | mass % | Liquid type | mass % |
| Reference Example 1 | ETMAH | 2.7 | | | | | | | | |
| Reference Example 2 | ETMAH | 7.3 | | | | | | | | |
| Reference Example 3 | ETMAH | 2.7 | | | | | | | | |
| Reference Example 4 | PTMAH | 3.1 | | | | | | | | |
| Reference Example 5 | BTMAH | 3.5 | | | | | | | | |
| Reference Example 6 | TEAH | 3.8 | | | | | | | | |
| Reference Example 7 | TPAH | 5.3 | | | | | | | | |
| Reference Example 8 | TBAH | 6.8 | | | | | | | | |
| Reference Example 9 | TBAH | 9.7 | | | | | | | | |
| Reference Example 10 | HTMAH | 8.3 | | | | | | | | |
| Reference Example 11 | DEDMA H | 3.1 | | | | | | | | |
| Reference Example 12 | ETMAH | 2 | PTMAH | 1 | | | | | | |
| Reference Example 13 | ETMAH | 1 | HTMAH | 5 | | | | | | |
| Reference Example 14 | ETMAH | 2.7 | | | SURFYNOL 465 | 20 | | | | |
| Reference Example 15 | PTMAH | 3.1 | | | SURFYNOL 465 | 20 | | | | |
| Reference Example 16 | BTMAH | 3.5 | | | SURFYNOL 465 | 20 | | | | |

**[Table 1-2]**

| | Hydroxi -de ion | Water | Halide ion | | Acute oral toxicity | Ecotoxicity (acute) | Development characteristics | Corrosion test | Halide ion/hydroxide ion |
|---|---|---|---|---|---|---|---|---|---|
| | mass% | mass % | Type | mass ppm | | | | | part by mass |
| Reference Example 1 | 0.44 | 97.3 | Cl | 0.03 | B | A | A | C | 0.0000069 |
| Reference Example 2 | 1.18 | 92.7 | Cl | 0.1 | B | A | A | C | 0.0000085 |
| Reference Example 3 | 0.44 | 97.3 | Cl | 210 | B | A | B | C | 0.0480933 |
| Reference Example 4 | 0.44 | 96.9 | Cl | 0.5 | B | A | A | C | 0.0001130 |
| Reference Example 5 | 0.44 | 96.5 | Cl | 0.3 | B | A | A | C | 0.0000682 |
| Reference Example 6 | 0.44 | 96.2 | Cl | 4 | A | A | A | C | 0.0009113 |
| Reference Example 7 | 0.44 | 94.7 | Br | 5 | A | A | A | C | 0.0011279 |
| Reference Example 8 | 0.44 | 93.2 | Br | 0.2 | A | A | A | C | 0.0000455 |
| Reference Example 9 | 0.64 | 90.3 | Br | 0.3 | A | A | A | C | 0.0000472 |
| Reference Example 10 | 0.44 | 91.7 | Cl | 13 | A | C | A | C | 0.0029465 |
| Reference Example 11 | 0.44 | 96.9 | Cl | 11 | B | A | A | C | 0.0024868 |
| Reference Example 12 | 0.47 | 97 | Cl | 0.1 | B | A | A | C | 0.0000215 |
| Reference Example 13 | 0.43 | 94 | Cl | 9 | A | C | A | C | 0.0021052 |
| Reference Example 14 | 0.44 | 97.3 | Cl | 0.03 | B | A | A | C | 0.0000068 |
| Reference Example 15 | 0.44 | 96.9 | Cl | 0.5 | B | A | A | C | 0.0001136 |
| Reference Example 16 | 0.44 | 96.5 | Cl | 0.3 | B | A | A | C | 0.0000682 |

**[Table 1-3]**

| | Quaternary ammonium hydroxide | | Quaternary ammonium hydroxide | | Surfactant | | Corrosion inhibitor | | Amine | |
|---|---|---|---|---|---|---|---|---|---|---|
| | Liquid type | mass % | Liquid type | mass% | Type | mass ppm | Liquid type | mass % | Liquid type | mass % |
| Example 1 | ETMAH | 2.7 | | | | | Benzotriazole | 1 | | |
| Example 2 | PTMAH | 3.1 | | | | | Benzotriazole | 1 | | |
| Example 3 | BTMAH | 3.5 | | | | | Benzotriazole | 1 | | |
| Example 4 | TEAH | 3.8 | | | | | Benzotriazole | 1 | | |
| Example 5 | TPAH | 5.3 | | | | | Benzotriazole | 1 | | |
| Example 6 | TBAH | 6.8 | | | | | Benzotriazole | 1 | | |
| Example 7 | TBAH | 9.7 | | | | | Benzotriazole | 1 | | |
| Example 8 | BTMAH | 3.5 | | | | | 2-butyne-1,4-diol | 1 | | |
| Example 9 | BTMAH | 3.5 | | | | | Catechol | 1 | | |
| Example 10 | ETMAH | 2.7 | | | SURFYNOL 465 | 20 | Benzotriazole | 1 | | |
| Example 11 | PTMAH | 3.1 | | | SURFYNOL 465 | 20 | Benzotriazole | 1 | | |
| Example 12 | BTMAH | 3.5 | | | SURFYNOL 465 | 20 | Benzotriazole | 1 | | |
| Example 13 | BTMAH | 3.5 | | | | | Benzotriazole | 1 | N,N-diethylethanolamine | 2 |
| Comparative Example 1 | TMAH | 2.4 | | | | | | | | |
| Comparative Example 2 | TMAH | 2.4 | | | SURFYNOL 465 | 20 | | | | |
| Comparative Example 3 | TMAH | 2.4 | | | | | Benzotriazole | 1 | | |

**[Table 1-4]**

| | Hydroxi -de ion | Water | Halide ion | | Acute oral toxicity | Ecotoxicity (acute) | Development characteristics | Corrosion test | Halide ion/hydroxide ion |
|---|---|---|---|---|---|---|---|---|---|
| | mass% | mass % | Type | mass ppm | | | | | part by mass |
| Example 1 | 0.44 | 96.3 | Cl | 0.1 | B | A | A | A | 0.0000229 |
| Example 2 | 0.44 | 95.9 | Cl | 0.6 | B | A | A | A | 0.0001356 |
| Example 3 | 0.44 | 95.5 | Cl | 0.4 | B | A | A | A | 0.0000909 |
| Example 4 | 0.44 | 95.2 | Cl | 3 | A | A | A | A | 0.0006835 |
| Example 5 | 0.44 | 93.7 | Br | 3 | A | A | A | A | 0.0006767 |
| Example 6 | 0.44 | 92.2 | Br | 0.3 | A | A | A | A | 0.0000682 |
| Example 7 | 0.64 | 89.3 | Br | 0.4 | A | A | A | A | 0.0000629 |
| Example 8 | 0.44 | 95.5 | Cl | 0.4 | B | A | A | A | 0.0000907 |
| Example 9 | 0.44 | 95.5 | Cl | 0.4 | B | A | A | A | 0.0000904 |
| Example 10 | 0.44 | 96.3 | Cl | 0.1 | B | A | A | A | 0.0000229 |
| Example 11 | 0.44 | 95.9 | Cl | 0.6 | B | A | A | A | 0.0001374 |
| Example 12 | 0.44 | 95.5 | Cl | 0.4 | B | A | A | A | 0.0000916 |
| Example 13 | 0.44 | 93.5 | Cl | 0.7 | B | A | A | A | 0.0001603 |
| Comparative Example 1 | 0.44 | 97.6 | Cl | 0.1 | C | C | A | C | 0.0000227 |
| Comparative Example 2 | 0.44 | 97.6 | Cl | 0.1 | C | C | A | C | 0.0000227 |
| Comparative Example 3 | 0.44 | 97.6 | Cl | 0.1 | C | C | A | B | 0.0000227 |

### Reference Signs List

1: Anode
2: Cathode
3: Power source
4: Anode chamber
5: Raw material chamber
6: Intermediate chamber
7: Cathode chamber
8: Anion-exchange membrane
9: Cation-exchange membrane

## Claims

1. An aqueous alkali solution, comprising the following (A) to (E):
(A) a quaternary ammonium ion represented by Formula (1) below;
(B) a halide ion;
(C) a hydroxide ion;
(D) water; and
(E) a corrosion inhibitor, 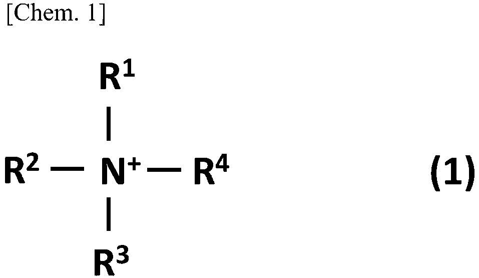 where R¹, R², R³, and R⁴ are each independently an alkyl group having carbon number from 1 to 16, with the proviso that the carbon number of one or more alkyl groups of R¹, R², R³, and R⁴ is from 2 to 16.

2. The aqueous alkali solution according to claim 1, wherein R¹, R², R³, and R⁴ in Formula (1) are not all the same alkyl group.

3. The aqueous alkali solution according to claim 1, wherein R¹, R², and R³ in Formula (1) are methyl groups, and R⁴ in Formula (1) is an alkyl group having carbon number from 2 to 16.

4. The aqueous alkali solution according to any one of claims 1 to 3, wherein the halide ion (B) is a chloride ion or a bromide ion.

5. The aqueous alkali solution according to any one of claims 1 to 4, wherein a part by mass of the halide ion is from 0.0000001 to 0.01 parts by mass when a mass of the hydroxide ion in the aqueous alkali solution is 1 part by mass.

6. The aqueous alkali solution according to any one of claims 1 to 5, wherein an LD50 value converted assuming that a hydroxide of the quaternary ammonium ion is taken as 100 mass%, in a toxicity test using a rat based on an acute oral toxicity test in accordance with OECD Test Guideline 420, is more than 50 mg/kg.

7. The aqueous alkali solution according to any one of claims 1 to 6, wherein an EC50 value converted assuming that a hydroxide of the quaternary ammonium ion is taken as 100 mass%, in a Daphnia sp. acute immobilisation test in accordance with OECD Test Guideline 202, is more than 3 mg/L.

8. The aqueous alkali solution according to any one of claims 1 to 7, further comprising one or more compounds selected from the group consisting of an alcohol and an amine.

9. The aqueous alkali solution according to any one of claims 1 to 8, further comprising a surfactant, wherein a concentration of the surfactant in the aqueous alkali solution is less than 100 mass ppm.

10. The aqueous alkali solution according to any one of claims 1 to 9, wherein the corrosion inhibitor (E) is one or more selected from the group consisting of an azole, an alkyne compound, and an aromatic hydroxy compound.

11. A method for using the aqueous alkali solution according to any one of claims 1 to 10 as a low toxic aqueous alkali solution for semiconductors.

12. A method for producing the aqueous alkali solution according to any one of claims 1 to 10, the method comprising an electrolysis step of electrolyzing an aqueous quaternary ammonium halide solution containing the following (A), (B), and (D), and a step of adding a corrosion inhibitor (E) to the aqueous solution obtained in the electrolysis step:
(A) a quaternary ammonium ion represented by Formula (1) below;
(B) a halide ion; and
(D) water 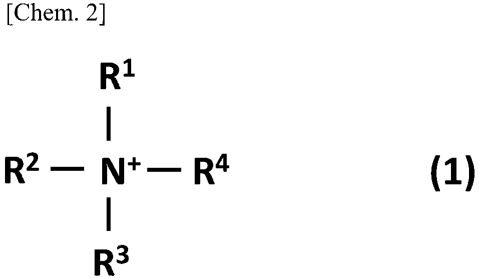
where R¹, R², R³, and R⁴ are each independently an alkyl group having carbon number from 1 to 16, with the proviso that the carbon number of one or more alkyl groups of R¹, R², R³, and R⁴ is from 2 to 16.

13. The method for producing the aqueous alkali solution according to claim 12, the method further comprising, before the electrolysis step, a preparation step of preparing the aqueous quaternary ammonium halide solution, wherein
the preparation step is a step of reacting a trialkylamine with an alkyl halide in ultrapure water to yield the aqueous quaternary ammonium halide solution.
